(19) Europäisches Patentamt
European Patent Office
Office européen des brevets

(11) Numéro de publication: **0 232 650 B1**

# (12) FASCICULE DE BREVET EUROPEEN

(45) Date de publication du fascicule du brevet: **13.12.89**

(51) Int. Cl.⁴: **C07K 7/10**, A61K 37/02 // (C07K7/10, 99:42),(C07K7/10, 99:46)

(21) Numéro de dépôt: **86402871.7**

(22) Date de dépôt: **19.12.86**

**(54) Nouveaux analogues de composés polypeptidiques hypocalcémiants épargnant le calcium de l'organisme, leur préparation, leur applicaton à titre de médicaments et les compositions les renfermant.**

(30) Priorité: **24.12.85 FR 8519113**

(43) Date de publication de la demande: **19.08.87 Bulletin 87/34**

(45) Mention de la délivrance du brevet: **13.12.89 Bulletin 89/50**

(84) Etats contractants désignés: **AT BE CH DE ES FR GB GR IT LI LU NL SE**

(56) Documents cités:
**EP-A- 0 146 842**
**EP-A- 0 181 121**
**FR-A- 2 309 235**
**FR-A- 2 521 553**
**US-A- 4 528 132**
**US-A- 4 537 716**

**Biochimica et Biophysica Acta, 322 (1973), pp. 383-391**

**Le dossier contient des informations techniques présentées postérieurement au dépôt de la demande et ne figurant pas dans le présent fascicule.**

(73) Titulaire: **MILHAUD, Gérard, 7, rue des Saints Pères, F-75006 Paris(FR)**

(72) Inventeur: **MILHAUD, Gérard, 7, rue des Saints Pères, F-75006 Paris(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 232 650 B1

ACTORUM AG

## Description

Dans la demande de brevet européen EP 0 146 842 est décrite la calcitonine de poulet. Cette calcitonine présente une activité similaire à la calcitonine de saumon et inférieure à celle des produits de la présente demande.

La présente invention a pour objet de nouveaux composés polypeptidiques, caractérisés en ce qu'ils sont choisis dans le groupe formé par les produits de formule ($I_A$) :

Cys – Asn – $A_3$ – Leu – Ser – Thr – Cys – $A_8$ – Leu – Gly –
$A_{11}$ – $A_{12}$ – $A_{13}$ – Gln – $A_{15}$ – $A_{16}$ – $A_{17}$ – Lys – ($I_A$)
$A_{19}$ – $A_{20}$ – Thr – $A_{22}$ – Pro – $A_{24}$ – Thr – $A_{26}$ – $A_{27}$ –
Gly – $A_{29}$ – Gly – $A_{31}$ – Pro $NH_2$

où : $A_3$ = Ser, Gly ou Ala
$A_8$ = Val ou Leu
$A_{11}$ = Lys ou Thr
$A_{12}$ = Leu ou Tyr
$A_{13}$ = Ser ou Thr
$A_{15}$ = Glu ou Asp
$A_{16}$ = Leu ou Phe
$A_{17}$ = His ou Asn
$A_{19}$ = Leu ou Phe
$A_{20}$ = Gln ou His
$A_{22}$ = Tyr ou Phe
$A_{24}$ = Arg ou Gln
$A_{26}$ = Asp, Asn ou Ala
$A_{27}$ = Val, Thr ou Ile.
$A_{29}$ = Ala, Ser ou Val
$A_{31}$ = Thr, Val ou Ala

et les produits de formule ($I_B$) qui contiennent l'acide α-aminosubérique (Asu) dont l'ω -carboxyle est fixé au groupe aminé de Asn :

$(CH_2)_5$
CO–Asn–A'$_2$–Leu–Ser–Thr–NHCHCO–A'$_7$–Leu–Gly–A'$_{10}$–
A'$_{11}$–A'$_{12}$–Gln–A'$_{14}$–A'$_{15}$–A'$_{16}$–Lys–A'$_{18}$– ($I_B$)
A'$_{19}$–Thr–A'$_{21}$–Pro–A'$_{23}$–Thr–A'$_{25}$–A'$_{26}$–Gly–
A'$_{28}$–Gly–A'$_{30}$–Pro $NH_2$

où : A'$_2$ = Ser, Gly ou Ala
A'$_7$ = Val ou Leu
A'$_{10}$ = Lys ou Thr
A'$_{11}$ = Leu ou Tyr
A'$_{12}$ = Ser ou Thr
A'$_{14}$ = Glu ou Asp
A'$_{15}$ = Leu ou Phe
A'$_{16}$ = His ou Asn
A'$_{18}$ = Leu ou Phe
A'$_{19}$ = Gln ou His
A'$_{21}$ = Tyr ou Phe
A'$_{23}$ = Arg ou Gln
A'$_{25}$ = Asp, Asn ou Ala
A'$_{26}$ = Val, Thr ou Ile
A'$_{28}$ = Ala, Ser ou Val
A'$_{30}$ = Thr, Val ou Ala

Les produits de formule ($I_B$) sont des analogues structuraux de peptides naturels, comme K. Jost et J. Rudinger les ont réalisés dans le cas des hormones neurohypophysaires (Collect. Czech. Chem. Comm., 1967, 32, 1229).

Les produits de formules ($I_A$) et ($I_B$) peuvent être regroupés dans la formule suivante :

D–A'$_7$–Leu–Gly–A'$_{10}$–A'$_{11}$–A'$_{12}$–Gln–A'$_{14}$–A'$_{15}$–A'$_{16}$–Lys–A'$_{18}$–A'$_{19}$–Thr–A'$_{21}$–Pro–A'$_{23}$–Thr–A'$_{25}$–A'$_{26}$–Gly–A'$_{28}$–Gly–A'$_{30}$–Pro $NH_2$

dans laquelle A'$_7$, A'$_{10}$, A'$_{11}$, A'$_{12}$, A'$_{14}$, A'$_{15}$, A'$_{16}$, A'$_{18}$, A'$_{19}$, A'$_{21}$, A'$_{23}$, A'$_{25}$, A'$_{26}$, A'$_{28}$ et A'$_{30}$ ont la signification précédente et D représente :

- soit le reste

Cys- Asn - $A_3$ - Leu - Ser - Thr - Cys -
dans lequel $A_3$ a la signification précédente
- soit le reste

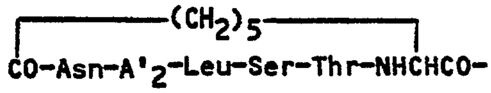

dans lequel $A'_2$ a la signification précédente.

L'invention a notamment pour objet les composés répondant aux formules générales ($I_A$) et ($I_B$), telles que définies précédemment, dans lesquelles $A_8$ et $A'_7$ représente une valine.

L'invention a plus particulièrement pour objet les composés répondant aux formules générales ($I_A$) et ($I_B$) suivantes :

- le Cys - Asn - Ser - Leu - $Ser_5$ - Thr - Cys - Val - Leu - $Gly_{10}$ - Lys - Leu - Ser - Gln - $Glu_{15}$ - Leu - His - Lys - Leu - $Gln_{20}$ - Thr - Tyr - Pro - Arg - $Thr_{25}$ - Asp - Val - Gly - Ala - $Gly_{30}$ - Thr - Proamide ;

- le 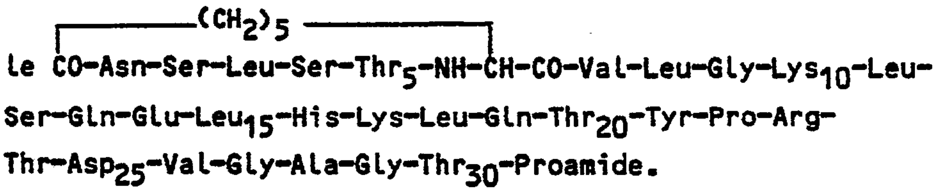

Les polypeptides selon l'invention abaissent la calcémie et la phosphatémie ; ils inhibent la destruction osseuse, accélèrent la formation d'os nouveaux, augmentent l'absorption intestinale du calcium et diminuent la calciurie. Ils fixent le calcium à l'intérieur des cellules. Ils possèdent un effet anti-inflammatoire et antalgique majeur.

Ces propriétés justifient l'utilisation des polypeptides selon l'invention, à titre de médicaments.

L'invention a donc également pour objet les polypeptides de formules ($I_A$) et ($I_B$) telles que définies précédemment, à titre de médicaments.

L'invention a plus particulièrement pour objet, à titre de médicaments, les polypeptides préférés mentionnés précédemment.

Les indications principales des médicaments selon l'invention sont représentées par la maladie de Paget, les ostéoporoses de diverses étiologies (commune, poro-malacique, cortisonique, post-traumatique, d'immobilisation et idiopathique), l'ostéodystrophie rénale, les fractures, les hypercalcémies, les douleurs ostéoarticulaires, en particulier celles consécutives aux métastases osseuses, la spasmophilie et la tétanie normocalcémique.

L'invention a également pour objet les compositions pharmaceutiques renfermant comme principe actif l'un au moins des médicaments tels que définis précédemment.

Ces compositions pharmaceutiques peuvent être, par exemple, solides ou liquides et se présenter sous les formes pharmaceutiques couramment utilisées en médecine humaine, comme, par exemple, les comprimés simples ou dragéifiés, les gélules, les capsules, les granulés, les liposomes, les aérosols, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a notamment pour objet les compositions pharmaceutiques telles que définies précédemment, caractérisés en ce qu'elles sont destinées à l'administration parentérale, orale sous forme de liposome ou nasale sous forme de pulvérisation.

L'invention a également pour objet un procédé de préparation des composés tels que définis précédemment, caractérisés en ce que l'on soumet des acides aminés, des peptides ou leurs combinaisons, à des réactions de condensation dans l'ordre de la séquence des acides aminés des formules ($I_A$) et ($I_B$) et par le fait que lors de la préparation des produits de formule ($I_B$), on associe la technique en phase solide pour l'obtention de la séquence 10-31 et la technique classique en phase liquide pour l'obtention de la séquence 1-9.

Comme indiqué précédemment, la synthèse des peptides de formule (IA) et celle de la séquence 10-31 des produits de formule ($I_B$) sont effectuées en utilisant la technique en phase solide. Celle-ci met en jeu la résine benzylhydrylaminée (P.G. Pietta et G.R. Marshall, Chem. Commun., 1970, 650), dont la préparation et l'utilisation ont été décrites (P. Rivaille, A. Robinson, M. Kamen et G. Milhaud, Helv., 1971, 54, 2772).

Dans un mode préférentiel de préparation des produits de formule ($I_A$), le groupe N-α-butyloxycarbonyle (t-Boc) protège la fonction α- aminée des aminoacides. Les fonctions latérales des aminoacides sont bloquées sous forme :

1) d'ester benzylique pour la chaîne carboxylque des acides aspartique et glutamique,
2) d'éther benzylique pour l'hydroxyle des sérine, thréonine et d'éther 2,4-dichlorobenzyle pour la tyrosine,
3) d'éther méthoxybenzylique pour le groupe sulfhydryle de la cystéine,
4) de dinitro-2,4-phényle pour l'imidazole de l'histidine,
5) de groupe benzoxycarbonyle pour la fonction ε-aminée de la lysine.

Les différents acides aminés sont incorporés dans la chaîne peptidique à l'aide de dicyclohexylcarbodiimide (DCCI) et d'hydroxybenzotriazole (HOBT) en faisant réagir successivement des aminoacides protégés en excès par rapport à la proline fixée en premier sur la résine. On laisse la réaction se poursuivre pendant deux heures. Les t-Boc sont éliminés par l'acide trifluoroacétique en solution dans le chlorure de méthylène ; après lavage au chlorure de méthylène, la neutralisation est effectuée par de la triéthylamine ou la diisopropyléthylamine (DIEA) dans ce même solvant.

L'asparagine et la glutamine sont introcuites sous forme de leurs esters paranitrophényliques, en solution dans le diméthylformamide (DMF). Si l'essai de contrôle du couplage à la ninhydrine suivant la méthode de Kaiser et Colescott est positif, on traite par de l'acide pivalique (1%) ; si la coloration bleue persiste encore dans cet essai, on sature le DMF/1% AcOH avec de l'urée. On peut cependant, même si ce test est négatif (pas de coloration bleue) répéter deux fois systématiquement chaque couplage. Si le couplage n'est pas total au bout de 48 heures de réaction, comme c'est le cas pour la glutamine en position 20, on acétyle la thréonine qui n'a pas réagi, par l'acétyl-imidazole dans le chlorure de méthylène ou par l'acide acétique et le DCCI.

A la fin, on élimine le groupe protecteur de l'imidazole de l'histidine, alors que le peptide est encore lié à la résine, à pH 8, avec du mercapto-éthanol en solution dans le DMF. Le peptide est séparé de la résine par traitement à l'acide fluorhydrique liquide en présence d'anisole et de méthionine. Tous les groupes protecteurs sont éliminés au cours de cette opération.

Les peptides sont purifiés par filtration sur gel. Les fractions correspondant aux pics principaux, sont rassemblées, lyophilisées, reprises dans un tampon à pH 8 et oxydées durant 24 heures par un courant d'air pour former le pont disulfure entre les deux résidus cystéiniques.

La fraction correspondant au pic possédant l'activité biologique recherchée est purifié à nouveau sur résine échangeuse d'ions CMC 32 à l'aide d'un gradient acide d'élution. Le peptide obtenu est homogène à l'électrophorèse sur papier et à la chromatographie sur cellulose.

La séquence 10-31 des produits de formule ($I_B$) est réalisée dans les mêmes conditions que celles indiquées précédemment pour la préparation des produits de formule ($I_A$). Ce que l'on a indiqué pour la glutamine en position 20 des produits de formule ($I_A$) vaut bien sûr pour la position 19 des produits de formule (IB).

Dans un mode préférentiel d'exécution du procédé de préparation des produits de formule ($I_B$), la synthèse de la séquence 1-9 est effectuée en milieu liquide. Le fragment

```
                                    (CH2)5COOR
                                     |
H-Asn-A'2-Leu-Ser-Thr-NH-CH-C=O
                                         |
                                        OH
```

où $A'_2$ a la signification ci-dessus et R = ester, après cyclisation, est condensé avec le tripeptide $A'_7$-Leu-Gly. On a recours aux groupes protecteurs généralement utilisés pour la synthèse des peptides.

Le Peptide de formule ($I_B$) est obtenu alors comme suit :

Le nonapeptide protégé ci-dessus est converti en ester N-hydroxysuccinimide puis couplé avec le fragment 10-31 fixé sur la résine en milieu DMF à 30¤C. Le peptide est séparé de la résine par traitement à l'acide fluorhydrique liquide en présence de diméthylsulfure et de p-crésol (J.P. Tam, W.H. Heathand et R.B. Merrifield, J.A.C.S., 1983, <u>105</u>, 6442). Tous les groupes protecteurs sont éliminés au cours de cette opération.

Les peptides sont purifiés par filtration sur gel. Les fractions correspondant aux pics principaux, sont rassemblées, lyophilisées. La fraction correspondant au pic possédant l'activité biologique recherchée est purifié à nouveau sur résine échangeuse d'ions CMC 32 par élution à l'aide d'un gradient de conductivité. Le peptide obtenu après lyophilisation apparaît homogène en électrophorèse sur papier et en chromatographie sur cellulose.

Les exemples suivants illustrent l'invention sans toutefois la limiter.

**Exemple 1** :

Obtention du peptide de formule ($I_A$) suivant :
Cys - Asn - Ser - Leu - $Ser_5$ - Thr - Cys - Val - Leu - $Gly_{10}$ - Lys - Leu - Ser - Gln - $Glu_{15}$ - Leu - His - Lys - Leu - $Gln_{20}$ - Thr - Tyr - Pro - Arg - $Thr_{25}$ - Asp - Val - Gly - Ala - $Gly_{30}$ - Thr - Proamide.($I_A$)

Les t-Boc aminoacides portant des fonctions latérales proviennent de BACHEM (Dubendorf, Suisse) ou sont préparés au laboratoire par la technique de Schnabel (E. Schnabel, Liebigs Ann. Chem., 702, 188, 1967) dans un autotitrateur. Leur pureté est déterminée par polarimétrie, point de fusion et chromatographie sur couche mince de silice.

L'hydrolyse du peptide purifié est réalisée en tube scellé sous vide en milieu HCl 6 N (110°C, 24 heures).

**I. SYNTHESE DE LA SEQUENCE.**

a) **Préparation de la résine** (P. Rivaille, A. Robinson, M. Kamen et G. Milhaud, Helv., 1971, 54, 2772).

. Benzyl-cétorésine.

Dans un ballon tricol de 2 l, muni d'un agitateur, on laisse gonfler pendant 2 heures, 50 g de polystyrène à 1% de divinylbenzène Bio-rad $SX_1$® (200-400 mesh) dans 350 ml de nitrobenzène. Dans un autre ballon, on dissout 66 g de $AlCl_3$ dans 300 ml de nitrobenzène auxquels on ajoute 80 ml de chlorure de benzoyle. Le mélange es versé dans le premier ballon sous bonne agitation maintenue pendant 2 heures à température ambiante. Si la résine gonfle trop, on ajoute du nitrobenzène (100 ml environ). On filtre, lave avec du dioxanne (3 x), de l'acide acétique (3 x), du méthanol (3 x) et enfin, alternativement, avec du $CH_2Cl_2$ et $CH_3OH$ pour faire gonfler et contracter la résine er laissant chaque fois un temps de contact suffisant. On sèche dans un dessicateur sous vide toute la nuit (rendement 75 g).

. Benzylhydrylamine résine.

Dans un ballon de 1 l muni de "Dean Stark" et d'un agitateur, on chauffe 500 g de formiate d'ammonium à 120-130°C, pendant 1 à 2 heures. On ajoute la résine (50 g) en une seule fois et laisse la température monter à 160-165°C, en une demi-heure, sous agitation efficace qui est maintenue pendant 6 heures. On lave à l'eau, puis on traite comme ci-dessus pour la benzylcétorésine. La résine séchée sous vide est chauffée à reflux et agitée pendant 8 heures dans HCl 6 N. Elle est ensuite filtrée et lavée comme précédemment. La capacité de fixation varie, selon la nature de l'acide aminé, entre 0,3 et 0,5 mmol/g.

b) **Préparation du peptide.**

L'appareil utilisé permet d'agiter mécaniquement un tube en pyrex de 25 ml dont le compartiment central contient la résine (2 g). Aux extrémités du tube, 2 filtres de verre fritté sont destinés à l'introduction puis à l'élimination des réactifs (environ 15 ml).

Le couplage des acides aminés comporte les temps suivants :

CYCLE 32

**Couplage :**

2 g de résine (capacité de fixation 0,5 mmol/g) sont agités (10 min) en milieu $CH_2Cl_2$, $(CH_3)_2NCHO$ (DMF) (1:1, v/v) (10 ml) en présence de Boc-L-proline (2mmol, 0,43 g). On ajoute DCCI (4 mmoles) et 1-hydroxybenzotriazole (HOBT) (4 mMoles) et on reprend l'agitation pendant 2 heures. La résine est lavée successivement avec $CH_2Cl_2$ (1x, 2min.), $CH_3OH$ abs (3x, 2mn.), $CH_2Cl_2$ (3x, 2min.). On effectue la réaction à la ninhydrine sur un échantillon de résine: elle est négative.

Libération du groupe aminé:

On traite la résine par le mélange $CF_3COOH$-$CH_2Cl_2$ (1:1, v/v) pendant 1min; on filtre et on répète l'opération en laissant 15min au contact. On lave la résine avec $CH_2Cl_2$ (3x, 1min). On élimine $CF_3COOh$ en traitant deux fois la résine par le mélange $Et_3N/CH_2Cl_2$ (12.5:87,5 v/v) (temps de contact 1 min puis 5 min). On lave avec $CH_2Cl_2$ (8x, 1min). La résine-L-proline est titrée: elle contient 0,5 mmol/g de proline.

CYCLE 31

Couplage:
On agite (10min) la résine L-prolyle (1mmol) avec 4 mmol de Boc-O-Benzyl-L-thréonine (1,23g) dans $CH_2Cl_2$-DMF (1:1 v/v) (10ml). On ajoute DCCI (4mmol) et HOBT (4 mmol) et on reprend l'agitation (2h). La résine est lavée successivement: $CH_2Cl_2$ (1x, 2min), $CH_3OH$ abs (3x, 2min), $CH_2Cl_2$ (3x, 2min). La réaction à la ninhydrine est négative.
Libération du groupe aminé – Voir cycle 32.

CYCLE 30 à 25

On utilise les procédés de couplage et de libération ci-dessus en faisant réagir pour:
. Cycle 30 1 mmol Boc-glycine (0,71g)
. Cycle 29 4 mmol Boc-L-ala (0,76g)
. Cycle 28 4 mmol Boc-glycine (0,71g)
. Cycle 27 4 mmol Boc-L-valine (0,87g)
. Cycle 26 4 mmol ester B-benzylique de l'acide Boc-L-aspartique (1,29g)
. Cycle 25 4 mmol Boc-O-benzyl-L-thréonine (1,24g).

CYCLE 24

Couplage:
On agite (10min) la résine-peptide du cycle 25, préalablement lavée avec DMF (2x), avec 4 mmol de Boc-N-γ-tosyl-L-arginine (1,41g) en solution de DMF et on procède comme décrit pour le cycle 31.

CYCLE 23

Couplage:
La résine peptide du cycle 24 est agitée (10min) avec 4mmol de Boc-L-proline (0,88g en milieu $CH_2Cl_2$ (10ml). On ajoute DCCI/HOBT comme décrit pour le cycle 32. A la fin de la période de réaction, la réaction à la ninhydrine est négative.

**Libération :** - Voir cycle 32.

**CYCLES 22-21**

On utilise les procédés de couplage et de libération utilisés dans le cycle 23 en faisant réagir :
. Cycle 22 4 mmol Boc-O-Benzyl-L-tyrosie (1,49 g)
. Cycle 21 4 mmol Boc-0-Benzyl-L-thréonine (1,24 g).

**CYCLE 20**

**Couplage :**

La résine-peptide du cycle 21, préalablement lavée avec DMF (2x) est agitée (10 min) avec 4 mmol de Boc-L-glutamine p-nitrophénylester (1,5 g) et 10 ml de DMF additionné de 1% d'acide acétique. On ajoute DCCI (4 mmol) HOBT (4 mmol) et on agite pendant 48 h. On lave la résine avec DMF, $CH_2Cl_2$, $CH_3OH$, $CH_2Cl_2$ (2x).

**Libération :** - Voir cycle 32.

**CYCLES 19 à 15**

On utilise le procédé du cycle 31 en faisant réagir :
. Cycle 19 4 mmol Boc-L-leucine (1,0 g)
. Cycle 18 4 mmol Boc-ε-carbobenzyloxy-L-lysine (1,52 g)
. Cycle 17 4 mmol Boc-dinitro-2,4-phényl(im))L-histidine (1,23 g)
. Cycle 16 4 mmol Boc-L-leucine (1,0 g)
. Cycle 15 4 mmol ester-γ-benzylique de l'acide Boc-L-glutamique (1,3 g).

**CYCLE 14**

On procède comme pour le cycle 20.

**CYCLE 13**

On procède comme pour le cycle 31 en utilisant 4 mmol de Boc-O-benzyl-L-sérine (1,0 g).

**CYCLES 12 à 9**

On procède comme pour le cycle 31 en faisant réagir :
. Cycle 12 : Le dérivé utilisé dans le cycle 19.
. Cycle 11 : Le dérivé utilisé dans le cycle 18.
. Cycle 10 : Le dérivé utilisé dans le cycle 30.
. Cycle 9 : Le dérivé utilisé dans le cycle 19.
. Cycle 8 : Le dérivé utilisé dans le cycle 27.

**CYCLE 7**

On utilise le procédé du cycle 31 en faisant réagir 4 mmol d'éther-S-p-méthoxybenzylique de Boc-L-cystéine (1,13 g).

**CYCLES 6 à 3**

. Cycle 6 : Identique au cycle 31.
. Cycle 5 : Identique au cycle 13.
. Cycle 4 : Identique au cycle 19.
. Cycle 3 : Identique au cycle 13.

**CYCLE 2**

**Couplage**

On agite (48 h) la résine peptide provenant du cycle 3 préalablement lavée avec DMF (2×), avec 4 mmol de p-nitrophényl ester de Boc-L-asparagine (1.45 g) en milieu DMF, en présence d'HOBT (4 mmol). La résine est lavée successivement avec DMF, $CH_2Cl_2$, $CH_3OH$, $CH_2Cl_2$. La réaction à la ninhydrine est négative.

**Libération**

On procède comme il est décrit pour le cycle 32.

**CYCLE 1**

On fait réagir dans les conditions du cycle 31, 4 mmol de l'éther S-p-méthoxybenzylique de la Boc-L-cystéine (1,13 g).

**II - ELIMINATION DU GROUPE DINITROPHENYLE DE L'HISTIDINE.**

A 15 ml de diméthylformamide (DMF), on ajoute 2 ml de mercaptoéthanol et on ajuste le pH à 8 avec de la triéthylamine. On introduit ce réactif dans l'appareil et on agite 14 h. On filtre et on lave la résine alternativement avec du chlorure de méthylène (3 ×), du méthanol (3 ×). Après séchage sous vide, le poids de la résine est de 2,85 g.

**III - LIBERATION DU PEPTIDE DE LA RESINE ET ELIMINATION DES GROUPES PROTECTEURS.**

On distille 15 ml d'acide fluorhydrique dans un mélange de 1,5 g de résine-peptide, 1,5 ml d'anisole. On agite 1 h à 0°C et 30 min à température ambiante. On chasse l'acide sous vide et on reprend le résidu dans 3 fois 5 ml d'acide acétique et on précipite par de l'éther éthylique exempt de péroxyde. On centrifuge à froid, on lave plusieurs fois le précipité à l'éther, on sèche sous vide. Le précipité est repris par de l'eau et le résidu insoluble éliminé par centrifugation. La solution lyophilisée fournit 800 mg de produit.

**IV - PURIFICATION DU PEPTIDE.**

Deux cents milligrammes de peptide brut sont placés au sommet d'une colonne (diamètre 2,5 cm, longueur 90 cm) de Biogel P6®, (50-100 mesh). On élue à l'aide d'acide acétique 0,1M et on recueille des fractions de 12 ml en suivant l'élution à l'aide d'un enregistreur 280 nm. Le peptide purifié est recueilli dans les fractions 23-28.

**V - CYCLISATION**

Les fractions contenant le peptide purifié sont lyophilisées et le résidu est repris dans 500 ml de tampon hydrogénocarbonate d'ammonium 1M, pH 8, et soumis à un barbotage d'air à travers une plaque poreuse pendant 24 h. La solution est lyophilisée et le résidu purifié sur colonne CMC 52 Whatman® par gradient d'acétate d'ammonium de mho 0,6 à mho 7, pH 4, avec un appareil LKB Ultrograd® 11300. Après hydrolyse acide, la composition en acides aminés est la suivante, rapportée à la proline = 2 (entre parenthèses les chiffres théoriques :
Ala (1) 1,05 ; Arg (1) 0,83 ; Asp (2) 2,10 ; Cys (2) 1,80 ; Glu (3) 3,10 ; Gly (3) 3,3 ; His (1) 0,80 ; Leu (5) 5,2 ; Lys (2) 1,90 ; Pro (2) 2,0 ; Ser (3) 3,10 ; Thr (4) 4,15 ; Tyr (1) 0,85 ; Val (2) 2,1.

**Exemple 2 :**

Obtention du peptide de formule (IB) suivant :

$$\overbrace{CO\text{-}Asn\text{-}Ser\text{-}Leu\text{-}Ser\text{-}Thr_5\text{-}NH\text{-}CH}^{(CH_2)_5}\text{-}CO\text{-}Val\text{-}Leu\text{-}Gly\text{-}Lys_{10}\text{-}Leu\text{-}$$
$$Ser\text{-}Gln\text{-}Glu\text{-}Leu_{15}\text{-}His\text{-}Lys\text{-}Leu\text{-}Gln\text{-}Thr_{20}\text{-}Tyr\text{-}Pro\text{-}Arg\text{-} \qquad (I_B)$$
$$Thr\text{-}Asp_{25}\text{-}Val\text{-}Gly\text{-}Ala\text{-}Gly\text{-}Thr_{30}\text{-}Proamide.$$

Les t-Boc aminoacides portant des fonctions latérales sont obtenus comme indiqué à l'exemple 1.
De même, l'hydrolyse du peptide purifié est réalisée en tube scellé sous vide en milieu HCl 6 N (110¤C, 24 h).

**1. SYNTHESE DE LA SEQUENCE 10-31 DU PEPTIDE.**

1-a/ **Préparation de la résine** (P. Rivaille, A. Robinson, M. Kamen et G. Milhaud, Helv., 1971, 54, 2772).

. Benzyl-cétorésine.

Dans un ballon tricol de 2 l, muni d'un agitateur, on laisse gonfler pendant 2 heures, 50 g de polystyrène à 1% de divinylbenzène Bio-rad $SX_1$® (200-400 mesh) dans 350 ml de nitrobenzène. Dans un autre ballon, on dissout 66 g de $AlCl_3$ dans 300 ml de nitrobenzène auxquels on ajoute 80 ml de chlorure de benzoyle. Le mélange est versé dans le premier ballon sous bonne agitation maintenue pendant 2 heures à température ambiante. Si la résine gonfle trop, on ajoute du nitrobenzène (100 ml environ). On filtre, lave avec du dioxanne (3 ×), de l'acide acétique (3 ×), du méthanol (3 ×) et enfin, alternativement, avec du $CH_2Cl_2$ et $CH_3OH$ pour faire gonfler et contracter la résine en laissant chaque fois un temps de contact suffisant. On sèche dans un dessicateur sous vide toute la nuit (rendement 75 g).

. Benzylhydrylamine résine.

Dans un ballon de 1 l muni de "Dean Stark" et d'un agitateur, on chauffe 500 g de formiate d'ammonium à 120-130¤C, pendant 1 à 2 heures. On ajoute la résine (50 g) en une seule fois et laisse la température monter à 160-165¤C, en une demi-heure, sous agitation efficace qui est maintenue pendant 6 heures. On filtre, lave à l'eau, puis on traite comme ci-dessus pour la benzylcétorésine. La résine séchée sous vide est chauffée à reflux et agitée pendant 8 heures dans HCl 6 N. Elle est ensuite filtrée et lavée comme précédemment. La capacité de fixation varie, selon la nature de l'acide aminé, entre 0,3 et 0,5 mmol/g.

1-b/ **Préparation de la séquence 10-31 du Peptide.**

L'appareil utilisé permet d'agiter mécaniquement un tube en pyrex de 25 ml dont le compartiment central contient a résine (2 g). Aux extrémités du tube, 2 filtres de verre fritté sont destinés à l'introduction puis à l'élimination des réactifs (environ 15 ml).

CYCLE 31

**Couplage :**

2 g de résine (capacité de fixation 0,5 mmol/g) sont agités (10 min) en milieu $CH_2Cl_2$, $(CH_3)_2NCHO$ (DMF) (1:1, v/v) (10 ml) en présence de Boc-L-proline (2mM, 0,43 g). On ajoute DCCI (4 mmol) et 1-hydroxybenzotriazole (HOBT) (4 mmol) et on reprend l'agitation pendant 2 heures. La résine est lavée successivement avec $CH_2Cl_2$ (1x, 2 min), $CH_3OH$ abs (3x, 2 min), $CH_2Cl_2$ (3x, 2min). On effectue la réaction à la ninhydrine sur un échantillon de résine : elle est négative.

**Libération du groupe aminé :**

On traite la résine par le mélange $CF_3CCOH$-$CH_2Cl_2$ (1:1, v/v) pendant 1 min; on filtre et on répète l'opération en laissant 15 min. au contact. On lave la résine avec $CH_2Cl_2$ (3x, 1 min). On élimine $CF_3COOH$ en traitant deux fois la résine par le mélange $Et_3N/CH_2Cl_2$ (12.5 : 87,5 v/v) (temps de contact 1 min puis 5 min). On lave avec $CH_2Cl_2$ (8x, 1 min). La résine-L-proline est titrée : elle contient 0,5 mmol/g de proline.

CYCLE 30

**Couplage :**

On agite (10 min) la résine L-prolyle (1mmol) avec 4 mMoles de Boc-O-Benzyl-L-thréonine (1,23 g) dans $CH_2Cl_2$ DMF (1:1 v/v) (10 ml). On ajoute DCCI (4 mmol) et HOBT (4 mmol) et on reprend l'agitation (2 h). La résine est lavée successivement : $CH_2Cl_2$ (1x, 2 min), $CH_3OH$ abs (3x, 2 min), $CH_2Cl_2$ (3x, 2 min). La réaction à la ninhydrine est négative.

**Libération du groupe aminé** - Voir cycle 31.

CYCLE 29 à 24

On utilise les procédés de couplage et de libération ci-dessus en faisant réagir pour :
. Cycle 29 4 mmol Boc-glycine (0,71 g)
. Cycle 28 4 mmol Boc-L-ala (0,76 g)
. Cycle 27 4 mmol Boc-glycine (0,71 g)
. Cycle 26 4 mmol Boc-L-valine (0,87 g)
. Cycle 25 4 mmol ester B-benzylique de l'acide Boc-L-aspartique (1,29 g)
. Cycle 24 4 mmol Boc-O-benzyl-L-thréonine (1,24 g).

CYCLE 23

**Couplage :**

On agite (10 min) la résine-peptide du cycle 24, préalablement lavée avec DMF (2x), avec 4 mmol de Boc-N-$\gamma$-tosyl-L-arginine (1,41 g) en solution de DMF et on procède comme décrit pour le cycle 30.

CYCLE 22

**Couplage :**

La résine peptide du cycle 23 est agitée (10 min) avec 4 mmol de Boc-L-proline (0,88 g en milieu $CH_2Cl_2$ (10 ml). On ajoute DCCI/HOBT comme décrit pour le cycle 31. A la fin de la période de réaction, la réaction à la ninhydrine était négative.

**Libération :** - Voir cycle 31.

CYCLES 21-20

On utilise les procédés de couplage et de libération utilisés dans le cycle 22 en faisant réagir :

. Cycle 21 4 mmol Boc-O-Benzyl-L-tyrosine (1,49 g)
. Cycle 20 4 mmol Boc-O-Benzyl-L-thréonine (1,24 g).

**CYCLE 19**

**Couplage :**

La résine-peptide du cycle 20, préalablement lavée avec DMF (2x) est agitée (10 min) avec 4 mmol de Boc-L-glutamine p-nitrophénylester (1,5 g) et 10 ml de DMF additionné de 1% d'acide acétique. On ajoute DCCI (4 mmol)-HOBT (4 mmol) et on agite pendant 48 h. On lave la résine avec DMF, $CH_2Cl_2$, $CH_3OH$, $CH_2Cl_2$ (2x).

**Libération :** - Voir cycle 31.

**CYCLES 18 à 14**

On utilise le procédé du cycle 30 en faisant réagir :
. Cycle 18 4 mmol Boc-L-leucine (1,0 g)
. Cycle 17 4 mmol Boc-ε-carbobenzyloxy-L-lysine (1,52 g)
. Cycle 16 4 mmol Boc-dinitro-2,4-phényl(im))L-histidine (1,23 g)
. Cycle 15 4 mmol Boc-L-leucine (1,0 g)
. Cycle 14 4 mmol ester-γ-benzylique de l'acide Boc-L-glutamique (1,3 g).

**CYCLE 13**

On procède comme pour le cycle 19.

**CYCLE 12**

On procède comme pour le cycle 30 en utilisant 4 mmol de Boc-O-benzyl-L-sérine (1,0 g).

**CYCLES 11 et 10**

On procède comme pour le cycle 30 en faisant réagir :
. Cycle 11 : Le dérivé utilisé dans le cycle 18.
. Cycle 10 : Le dérivé utilisé dans le cycle 17.

**2. SYNTHESE DE LA SEQUENCE 1-9 DU PEPTIDE.**

2-a/ **Séquence partielle BOC-L-Thr (Bzl)-Asu OMe (A).**

On introduit Z-Asu OMe (11,2 g) dans MeOH-$H_2O$ (2:1, v/v) et on hydrogène en présence de charbon palladié (14 h.). Le catalyseur éliminé, on concentre sous vide. On ajoute du dioxanne (40 ml), puis la triéthylamine (4,2 ml) en refroidissant puis le BOC-L-Thr (Bzl) OSu (16 g). Après agitation (72 h), on introduit de la N,N-diméthylamino-1,3-propane diamine, agite (4 h), concentre au tiers. Extraction avec acétate d'éthyle (AcOEt). Lavage de AcOEt avec HCl (1 N) puis $H_2O$. Distillation sous vide. Reprise du résidu huileux dans l'éther, extraction avec $NaHCO_3$ (5%). Réextraction avec AcOEt et lavages successifs avec $H_2O$, HCl (1 N), $H_2O$. Séchage sur sulfate de sodium anhydre. AcOEt est chassé. On obtient A sous forme de produit huileux (10 g).

**2-b/ Séquence partielle BOC-L-Ser (Bzl)-L-Thr (Bzl) Asu OMe.CHA (B).**

A (5 g) est dissous en refroidissant dans TFA (15 ml). A température ambiante, TFA est chassé sous vide et le résidu séché sous vide. Il est dissous dans DMF (10 ml) et le pH amené à 6 à l'aide de triéthylamine (4 ml) en refroidissant. On ajoute alors HOBT (5 g) et BOC-L-Ser (Bzl) OSu et on agite (3 jours) à température ambiante, à pH 6. On ajoute de la N,N-diméthylamino-1,3-propanediamine, agite (1 h), ajoute $H_2O$, extrait par AcOEt comme décrit en ci-dessus. La cyclohexylamine (CHA) est ajoutée à AcOEt séché. Distillation sous pression réduite. On obtient le produit huileux B (4 g).

**2-c/ Séquence partielle BOC-L-Asn-L-Ser(Bzl)-L-Leu-NH-NH@ (C).**

On dissout 3 g de BOC-L-Asn-L-Ser(Bzl)-L-Leu OEt dans 20 ml de $CH_3OH$ et ajoute 10 ml de $NH_2$-

$NH_2$-$H_2O$ à 80%. On abandonne pendant 14 h à la température ambiante. On précipite à l'éther éthylique, lave à l'éther éthylique et recristallise dans le mélange $CH_3OH$-AcOEt-éther éthylique. On obtient 2,5 g de produit attendu (C).

**2d/ Séquence partielle BOC-L-Asn-L-Ser (Bzl)-L-Leu-L-Ser (Bzl)-L-Thr (Bzl)-Asu OMe (D).**

B (2 g) est introduit dans AcOEt en présence de HCl (1 N) ; séchage sur sulfate de sodium anhydre et concentration sous vide. Addition à froid de TFA (6 ml), agitation à température ambiante (0,5h), élimination du TFA sous vide, séchage du résidu sous vide. Dissolution du résidu dans DMF (2 ml), neutralisation avec triéthylamine puis introduction lente à -40°C dans une solution de C BOC-L-Asn-L-Ser(Bzl)-L-Leu-NH-$NH_2$ (1,7 g) dans DMF (6 ml), à laquelle on a préalablement ajouté dioxanne (2,8 ml) en présence HCl (1 N) et nitrite d'isoamyle (0,6 ml). Ajustement du pH à 7 avec triéthyl-amine ; réaction à 5°C (72 h).

Introduction lente du mélange réactionnel à -5°C dans HCl 0,5 N (60 ml). Lavage du précipité par $H_2O$. Extraction par $CHCl_3$ (100 ml) ; lavages successifs avec HCl (1 N) NaCl aqueux. Elimination du $CHCl_3$. Précipitation de D par le mélange $CHCl_3$-n hexane (2 g).

**2-e/ Cyclisation -L-Asn-L-Ser (Bzl)-L-Leu-L-Ser (Bzl)-L-Thr (Bzl) Asu1,7-NH-NH@ (E).**

Dissolution de D (1,6 g) dans pyridine anhydre (15 ml). Addition de TFA-ONP (2,5 g). Agitation à 45°C (3 h). Concentration sous vide. Précipitation par éther éthylique. Traitement par TFA comme ci-dessus. Cyclisation dans la pyridine anhydre à 50°C pendant 5 h. Extraction par $CHCl_3$ et lavage comme décrit ci-dessus. Concentration sous vide et précipitation par n-hexane.

Dissolution du précipité (2 g) dans DMF (5 ml) et $CH_3OH$ (25 ml). Addition de 15 ml d'hydrazine (80%). Agitation à température ambiante (14h). Addition de $H_2O$. Filtration du précipité. Lavage à $H_2O$ Addition de $CH_3OH$ (50ml) et chauffage à reflux. Précipitation de E (0,8 g).

**2-f/ Préparation de la séquence 1-9 : -L-Asn-L-Ser (Bzl)-L-Leu-L-Ser (Bzl)-L-Thr (Bzl)-Asu1,7-L-Val-L-Leu-Gly OH (F).**

E (1 g) est en suspension dans DMF (4 ml), additionné de dioxanne HCl 4 N (1,7 ml) à -5°C. Ajout de nitrite d'isoamyle (0,3 ml) à -10°ous agitation. Addition de L-H-Val-L-Leu-Gly (0,9 g) à -5°C, le pH étant amené à 7 par triéthylamine. Agitation en bain glacé (48 h). Précipitation par HCl 0,5 N (150 ml) de F (1 g).

**3. PREPARATION DU PEPTIDE.**

3-a/ F (500 mg) dissous dans DMF (2 ml) est additionné de HOBT (111 mg) et DCCI (150 mg). Agitation pendant 72 h en présence du peptidyl résine 10-31 (300 mg) après avoir éliminé le t-BOC de $Lys_{10}$.

**3-b/ Clivage du peptide** selon Tam et al. JACS (1983), 105, 6442.

Les quantités sont rapportées à 1 g de peptidyl résine.

1) On distille HF (2,5 ml) dans le mélange résine-peptide (1 g), diméthylsulfure (6,5 ml), p-crésol (250 mg). Agitatior (1 h) à 0°C. On chasse l'acide et le diméthylsulfure sous vide et on reprend le résidu par 3 fois 5 ml d'AcOEt.

2) Le peptidyl résine séché, suspendu dans le diméthyl-sulfure (1 ml) est traité par HF liquide (10 ml) à 0°C (1 h). Après élimination du HF et du diméthylsulfure sous vide, la résine est lavée à l'éther (3 fois 10 ml) ; le peptide est repris à l'acide acétique et précipité à l'éther éthylique. On centrifuge à froid, on lave plusieurs fois le précipité à l'éther, on sèche sous vide. Le précipité est repris par de l'eau et le résidu insoluble éliminé par centrifugation. La solution lyophilisée fournit 500 mg de produit.

**3-c/ Purification du peptide.**

Deux cent milligrammes de peptide brut sont placés au sommet d'une colonne (diamètre 2,5 cm, longueur 90 cm) de Biogel® P6, 50-100 mesh. On élue à l'aide d'acide acétique 0,1M et on recueille des fractions de 12 ml en suivant l'élution à l'aide d'un enregistreur 280 nm. Le peptide purifié est recueilli dans les fractions 23-28. Ces fractions sont lyophilisées et le résidu purifié sur colonne CMC 32 Whatman® par gradient d'acétate d'ammonium du mho 0,6 à mho 7, pH 4, avec un appareil LKB Ultrograd® 11300.

Après hydrolyse acide, la composition en acides aminés est la suivante, rapportée à la proline = 2 (entre parenthèses les chiffres théoriques) :
Ala (1) 1,15 ; Arg (1) 0,85 ; Asp (2) 2,15 ; Glu (3) 3,10 ; Gly (3) 2,9 ; His (1) 0,83 ; Leu (5) 5,3 ; Lys (2) 1,95 ; Pro (2) 2,0 ; Ser (3) 3,20 ; Thr (4) 4,10 ; Tyr (1) 0,80 ; Val (2) 2,1 ; Asu (1) 0,93.

Abréviations : AcOEt= Acétate d'éthyle
Asu= Acide α-aminosubérique
Bzl= Benzyle
CHA= Cyclohexylamine
DCHA= Dicyclohexylamine
ONP= Ester p-nitrophénylique
OSu= Ester N-hydroxysuccinimide
Z= Benzyloxycarbonyle

**- ACTIVITE BIOLOGIQUE DES PRODUITS DES EXEMPLES.**

Elle est déterminée chez le rat mâle pesant de 100 à 120 g et soumis à un jeûne de 16 heures. L'activité est exprimée en unité MRC à partir de la baisse de la calcémie, mesurée une heure après l'injection intraveineuse de peptide à tester convenablement dilué en tampon acétate de sodium 0,1 N pH 6 et 0,1% d'albumine, par comparaison avec l'activité du "Research Standard B" convenablement dilué. L'activité biologique du peptide de l'exemple 1 est supérieure à 4000 unités MRC par mg et celle du peptide de l'exemple 2 est supérieure à 4500 unités MRC par mg.

Compte tenu de cette activité, la dose usuelle, variable selon le produit utilisé, le sujet traité et l'affection en cause peut être par exemple de 1 à 100 unités MRC par jour par voie intramusculaire ou sous cutanée.

**Revendications pour les Etats désignés BE, CH, DE, FR, GB, IT, LI, LU, NL, SE**

1.- Nouveaux polypeptidiques, caractérisés er ce qu'ils sont choisis dans le groupe formé par les produits de formule ($I_A$) :

Cys - Asn - $A_3$ - Leu - Ser - Thr - Cys - $A_8$ - Leu -Gly - $A_{11}$ - $A_{12}$ - $A_{13}$ - Gln - $A_{15}$ - $A_{16}$ - $A_{17}$ - Lys - $A_{19}$ - $A_{20}$ - Thr -$A_{22}$ - Pro - $A_{24}$ - Thr - $A_{26}$ - $A_{27}$ - Gly - $A_{29}$ - Gly - $A_{31}$ - Pro $NH_2$($I_A$)

où :

$A_3$ = Ser, Gly, ou Ala
$A_8$ = Val ou Leu
$A_{11}$ = Lys ou Thr
$A_{12}$ = Leu ou Tyr
$A_{13}$ = Ser ou Thr
$A_{15}$ = Glu ou Asp
$A_{16}$ = Leu ou Phe
$A_{17}$ = His ou Asn
$A_{19}$ = Leu ou Phe
$A_{20}$ = Gln ou His
$A_{22}$ = Tyr ou Phe
$A_{24}$ = Arg ou Gln
$A_{26}$ = Asp, Asn ou Ala
$A_{27}$ = Val, Thr ou Ile
$A_{29}$ = Ala, Ser ou Val
$A_{31}$ = Thr, Val ou Ala

et les produits de formule ($I_B$):

$(CH_2)_5$
CO-Asn-$A'_2$-Leu-Ser-Thr-NHCHCO-$A'_7$-Leu-Gly-$A'_{10}$-
$A'_{11}$-$A'_{12}$-Gln-$A'_{14}$-$A'_{15}$-$A'_{16}$-Lys-$A'_{18}$- ($I_B$)
$A'_{19}$-Thr-$A'_{21}$-Pro-$A'_{23}$-Thr-$A'_{25}$-$A'_{26}$-Gly-
$A'_{28}$-Gly-$A'_{30}$-Pro $NH_2$

où :

$A'_2$ = Ser, Gly ou Ala
$A'_7$ = Val ou Leu
$A'_{10}$ = Lys ou Thr
$A'_{11}$ = Leu ou Tyr
$A'_{12}$ = Ser ou Thr
$A'_{14}$ = Glu ou Asp

$A'_{15}$ = Leu ou Phe
$A'_{16}$ = His ou Asn
$A'_{18}$ = Leu ou Phe
$A'_{19}$ = Gln ou His
$A'_{21}$ = Tyr ou Phe
$A'_{23}$ = Arg ou Gln
$A'_{25}$ = Asp, Asn ou Ala
$A'_{26}$ = Val, Thr ou Ile
$A'_{28}$ = Ala, Ser ou Val
$A'_{30}$ = Thr, Val ou Ala

2.- Nouveaux polypeptides répondant à la formule ($I_A$) telle que définie à la revendication 1 dans laquelle le symbole $A_8$ représente une valine.

3.- Nouveaux polypeptides répondant à la formule (IB) telle que définie à la revendication 1 dans laquelle le symbole $A'_7$ représente une valine.

4.- Le produit de formule ($I_A$) selon la revendication 1 suivant :
Cys - Asn - Ser - Leu - $Ser_5$ - Thr - Cys - Val - Leu - $Gly_{10}$- Lys - Leu - Ser - Gln - $Glu_{15}$ - Leu - His - Lys - Leu - $Gln_{20}$ - Thr - Tyr - Pro - Arg - $Thr_{25}$ - Asp - Val - Gly - Ala - $Gly_{30}$ - Thr - Proamide.($I_A$)

5.- Le produit de formule ($I_B$) selon la revendication 1 suivant :

$(CH_2)_5$ (pont reliant CO et CH)
CO-Asn-Ser-Leu-Ser-$Thr_5$-NH-CH-CO-Val-Leu-Gly-$Lys_{10}$-Leu-
Ser-Gln-Glu-$Leu_{15}$-His-Lys-Leu-Gln-$Thr_{20}$-Tyr-Pro-Arg- ($I_B$)
Thr-$Asp_{25}$-Val-Gly-Ala-Gly-$Thr_{30}$-Proamide.

6.- Procédé de préparation des nouveaux polypeptides spécifiés dans les revendications 1 à 5, caractérisé par le fait que l'on soumet des acides aminés, des peptides ou leurs combinaisons, à des réactions de condensation dans l'ordre de la séquence des acides aminés des formules ($I_A$) et ($I_B$) et par le fait que lors de la préparation des produits de formule ($I_B$) on associe la technique en phase solide pour l'obtention de la séquence 10-31 et la technique classique en phase liquide pour l'obtention de la séquence 1-9.

7.- A titre de médicament, les polypeptides mentionnés dans les revendications 1 à 5.

8.- Compositions pharmaceutiques contenant comme principe actif un médicament selon la revendication 7.

9.- Les préparations pharmaceutiques contenant comme principe actif un ou plusieurs polypeptides mentionnés dans les revendications 1 à 5, ainsi que les véhicules adéquats destinés à l'administration parentérale, orale sous forme de liposome et nasale sous forme de pulvérisation.

**Revendications pour l'Etat contractants: AT, ES**

1.- Procédé de préparation des nouveaux polypeptides, choisis dans le groupe formé par les produits de formule ($I_A$) :
Cys - Asn - $A_3$ - Leu - Ser - Thr - Cys - $A_8$- Leu -Gly - $A_{11}$ - $A_{12}$ - $A_{13}$ - Gln - $A_{15}$ - $A_{16}$ - $A_{17}$ - Lys - $A_{19}$ - $A_{20}$ - Thr -$A_{22}$ - Pro - $A_{24}$ - Thr - $A_{26}$ - $A_{27}$ - Gly - $A_{29}$ - Gly - $A_{31}$ - Pro $NH_2$($I_A$)
où :
$A_3$ = Ser, Gly ou Ala
$A_8$ = Val ou Leu
$A_{11}$ = Lys ou Thr
$A_{12}$ = Leu ou Tyr
$A_{13}$ = Ser ou Thr
$A_{15}$ = Glu ou Asp
$A_{16}$ = Leu ou Phe
$A_{17}$ = His ou Asn
$A_{19}$ = Leu ou Phe
$A_{20}$ = Gln ou His
$A_{22}$ = Tyr ou Phe
$A_{24}$ = Arg ou Gln
$A_{26}$ = Asp, Asn ou Ala
$A_{27}$ = Val, Thr ou Ile
$A_{29}$ = Ala, Ser ou Val
$A_{31}$ = Thr, Val ou Ala
et les produits de formule ($I_B$) :

(CH2)5 ┐

CO-Asn-A'$_2$-Leu-Ser-Thr-NHCHCO-A'$_7$-Leu-Gly-A'$_{10}$-
A'$_{11}$-A'$_{12}$-Gln-A'$_{14}$-A'$_{15}$-A'$_{16}$-Lys-A'$_{18}$-
A'$_{19}$-Thr-A'$_{21}$-Pro-A'$_{23}$-Thr-A'$_{25}$-A'$_{26}$-Gly-
A'$_{28}$-Gly-A'$_{30}$-Pro $NH_2$ ($I_B$)

où :

A'$_2$ = Ser, Gly ou Ala
A'$_7$ = Val ou Leu
A'$_{10}$ = Lys ou Thr
A'$_{11}$ = Leu ou Tyr
A'$_{12}$ = Ser ou Thr
A'$_{14}$ = Glu ou Asp
A'$_{15}$ = Leu ou Phe
A'$_{16}$ = His ou Asn
A'$_{18}$ = Leu ou Phe
A'$_{19}$ = Gln ou His
A'$_{21}$ = Tyr ou Phe
A'$_{23}$ = Arg ou Gln
A'$_{25}$ = Asp, Asn ou Ala
A'$_{26}$ = Val, Thr, ou Ile
A'$_{28}$ = Ala, Ser ou Val
A'$_{30}$ = Thr, Val ou Ala

caractérisé par le fait que l'on soumet des acides aminés, des peptides ou leurs combinaisons, à des réactions de condensation dans l'ordre de la séquence des acides aminés des formules ($I_A$) et ($I_B$) et par le fait que lors de la préparation des produits de formule ($I_B$) on associe la technique en phase solide pour l'obtention de la séquence 10-31 et la technique classique en phase liquide pour l'obtention de la séquence 1-9.

2.- Procédé selon la revendication 1, caractérisé en ce que dans la formule ($I_A$), le symbole $A_8$ représente une valine et en ce que dans la formule ($I_B$), le symbole A'$_7$ représente une valine.

3.- Procédé selon la revendication 1 ou 2, caractérisé en ce que les produit de départ sont choisis de manière telle que l'on prépare le produit de formule ($I_A$) selon la revendication 1 suivant :

Cys - Asn - Ser - Leu - Ser$_5$- Thr - Cys - Va - Leu - Gly$_{10}$ - Lys - Leu - Ser - Gln - Glu$_{15}$ - Leu - His - Lys - Leu - Gln$_{20}$ - Thr - Tyr - Pro - Arg - Thr$_{25}$ - Asp - Val - Gly - Ala - Gly$_{30}$- Thr - Proamide.($I_A$)

4.- Procédé selon la revendication 1 ou 2, caractérisé en ce que les produits de départ sont choisis de manière telle que l'on prépare le produit de formule ($I_B$) selon la revendication 1 suivant :

(CH2)5 ┐

CO-Asn-Ser-Leu-Ser-Thr$_5$-NH-CH-CO-Val-Leu-Gly-Lys$_{10}$-Leu-
Ser-Gln-Glu-Leu$_{15}$-His-Lys-Leu-Gln-Thr$_{20}$-Tyr-Pro-Arg-
Thr-Asp$_{25}$-Val-Gly-Ala-Gly-Thr$_{30}$-Proamide. ($I_B$)

**Revendications pour l'Etat contractant: GR**

1.- Procédé de préparation des nouveaux polypeptides, choisis dans le groupe formé par les produits de formule ($I_A$) :

Cys - Asn - $A_3$ - Leu - Ser - Thr - Cys - $A_8$ - Leu -Gly - $A_{11}$ - $A_{12}$ - $A_{13}$ - Gln - $A_{15}$ - $A_{16}$ - A $_{17}$ - Lys - $A_{19}$ - $A_{20}$ - Thr -$A_{22}$ - Pro - $A_{24}$ - Thr - $A_{26}$ - $A_{27}$ - Gly - $A_{29}$ - Gly - $A_{31}$ - Pro $NH_2$($I_A$)

où :

$A_3$ = Ser, Gly ou Ala
$A_8$ = Val ou Leu
$A_{11}$ = Lys ou Thr
$A_{12}$ = Leu ou Tyr
$A_{13}$ = Ser ou Thr
$A_{15}$ = Glu ou Asp

$A_{16}$ = Leu ou Phe
$A_{17}$ = His ou Asn
$A_{19}$ = Leu ou Phe
$A_{20}$ = Gln ou His
$A_{22}$ = Tyr ou Phe
$A_{24}$ = Arg ou Gln
$A_{26}$ = Asp, Asn ou Ala
$A_{27}$ = Val, Thr ou Ile
$A_{29}$ = Ala, Ser ou Val
$A_{31}$ = Thr, Val ou Ala
et les produits de formule ($I_B$) :

$$\overline{\text{CO-Asn-A}'_2\text{-Leu-Ser-Thr-NHCHCO}}^{(CH_2)_5}\text{-A}'_7\text{-Leu-Gly-A}'_{10}\text{-}$$
$$\text{A}'_{11}\text{-A}'_{12}\text{-Gln-A}'_{14}\text{-A}'_{15}\text{-A}'_{16}\text{-Lys-A}'_{18}\text{-} \qquad (I_B)$$
$$\text{A}'_{19}\text{-Thr-A}'_{21}\text{-Pro-A}'_{23}\text{-Thr-A}'_{25}\text{-A}'_{26}\text{-Gly-}$$
$$\text{A}'_{28}\text{-Gly-A}'_{30}\text{-Pro NH}_2$$

où :
$A'_2$ = Ser, Gly ou Ala
$A'_7$ = Val ou Leu
$A'_{10}$ = Lys ou Thr
$A'_{11}$ = Leu ou Tyr
$A'_{12}$ = Ser ou Thr
$A'_{14}$ = Glu ou Asp
$A'_{15}$ = Leu ou Phe
$A'_{16}$ = His ou Asn
$A'_{18}$ = Leu ou Phe
$A'_{19}$ = Gln ou His
$A'_{21}$ = Tyr ou Phe
$A'_{23}$ = Arg ou Gln
$A'_{25}$ = Asp, Asn ou Ala
$A'_{26}$ = Val, Thr ou Ile
$A'_{28}$ = Ala, Ser ou Val
$A'_{30}$ = Thr, Val ou Ala
caractérisé par le fait que l'on soumet des acides aminés, des peptides ou leurs combinaisons, à des réactions de condensation dans l'ordre de la séquence des acides aminés des formules ($I_A$) et ($I_B$) et par le fait que lors de la préparation des produits de formule ($I_B$) on associe la technique en phase solide pour l'obtention de la séquence 10-31 et la technique classique en phase liquide pour l'obtention de la séquence 1-9.

2.- Procédé selon la revendication 1, caractérisé en ce que dans la formule ($I_A$), le symbole $A_8$ représente une valine et en ce que dans la formule ($I_B$), le symbole $A'_7$ représente une valine.

3.- Procédé selon la revendication 1 ou 2, caractérisé en ce que les produit de départ sont choisis de manière telle que l'on prépare le produit de formule ($I_A$) selon la revendication 1 suivant :
Cys - Asn - Ser - Leu - $Ser_5$ - Thr - Cys - Val - Leu - $Gly_{10}$ - Lys - Leu - Ser - Gln - $Glu_{15}$ - Leu - His - Lys - Leu - $Gln_{20}$ - Thr - Tyr - Pro - Arg - $Thr_{25}$ - Asp - Val - Gly - Ala - $Gly_{30}$ - Thr - Proamide.($I_A$)

4.- Procédé selon la revendication 1 ou 2, caractérisé en ce que les produits de départ sont choisis de manière telle que l'on prépare le produit de formule ($I_B$) selon la revendication 1 suivant :

$$\overline{\text{CO-Asn-Ser-Leu-Ser-Thr}_5\text{-NH-CH}}^{(CH_2)_5}\text{-CO-Val-Leu-Gly-Lys}_{10}\text{-Leu-}$$
$$\text{Ser-Gln-Glu-Leu}_{15}\text{-His-Lys-Leu-Gln-Thr}_{20}\text{-Tyr-Pro-Arg-} \qquad (I_B)$$
$$\text{Thr-Asp}_{25}\text{-Val-Gly-Ala-Gly-Thr}_{30}\text{-Proamide.}$$

5.- Procédé de préparation de compositions pharmaceutiques, caractérisé en ce que l'on met, à titre de principe actif, l'un au moins des polypeptides de formule ($I_A$) ou ($I_B$), telles que définies à la revendication 1, sous une forme destinée à cet usage.

6.- Procédé de préparation de compositions pharmaceutiques, caractérisé en ce que l'on met, à titre de principe actif, l'un au moins des polypeptides de formule ($I_A$) ou ($I_B$), telles que définies à la revendication 3 ou 4, sous une forme destinée à cet usage.

**Patentansprüche für die Vertragsstaaten: BE, CH, DE, FR, GB, IT, LI, LU, NL, SE**

1. Neue Polypeptide, dadurch gekennzeichnet, daß sie unter den Produkten der Formel ($I_A$)
Cys–Asn–$A_3$–Leu–Ser–Thr–Cys–$A_8$–Leu–Gly–$A_{11}$–$A_{12}$–$A_{13}$–Gln–$A_{15}$–$A_{16}$–$A_{17}$–Lys–$A_{19}$–$A_{20}$–Thr–$A_{22}$–Pro–$A_{24}$–Thr–$A_{26}$–$A_{27}$–Gly–$A_{29}$–Gly–$A_{31}$–Pro $NH_2$ ($I_A$)
worin
$A_3$=Ser, Gly oder Ala
$A_8$=Val oder Leu
$A_{11}$=Lys oder Thr
$A_{12}$=Leu oder Tyr
$A_{13}$=Ser oder Thr
$A_{15}$=Glu oder Asp
$A_{16}$=Leu oder Phe
$A_{17}$=His oder Asn
$A_{19}$=Leu oder Phe
$A_{20}$=Gln oder His
$A_{22}$=Tyr oder Phe
$A_{24}$=Arg oder Gln
$A_{26}$=Asp, Asn oder Ala
$A_{27}$=Val, Thr oder Ile
$A_{29}$=Ala, Ser oder Val
$A_{31}$=Thr, Val oder Ala,
und den Produkten der Formel ($I_B$)

$(CH_2)_5$
CO–Asn–$A'_2$–Leu–Ser–Thr–NHCHCO–$A'_7$–Leu–Gly–$A'_{10}$–
$A'_{11}$–$A'_{12}$–Gln–$A'_{14}$–$A'_{15}$–$A'_{16}$–Lys–$A'_{18}$–
$A'_{19}$–Thr–$A'_{21}$–Pro–$A'_{23}$–Thr–$A'_{25}$–$A'_{26}$–Gly–
$A'_{28}$–Gly–$A'_{30}$–Pro $NH_2$ ($I_B$)

worin
$A'_2$=Ser, Gly oder Ala
$A'_7$=Val oder Leu
$A'_{10}$=Lys oder Thr
$A'_{11}$=Leu oder Tyr
$A'_{12}$=Ser oder Thr
$A'_{14}$=Glu oder Asp
$A'_{15}$=Leu oder Phe
$A'_{16}$=His oder Asn
$A'_{18}$=Leu oder Phe
$A'_{19}$=Gln oder His
$A'_{21}$=Tyr oder Phe
$A'_{23}$=Arg oder Gln
$A'_{25}$=Asp, Asn oder Ala
$A'_{26}$=Val, Thr oder Ile
$A'_{28}$=Ala, Ser oder Val
$A'_{30}$=Thr, Val oder Ala,
ausgewählt sind.

2. Neue Polypeptide der Formel ($I_A$) gemäß Anspruch 1, worin das Symbol $A_8$ für ein Valin steht.

3. Neue Polypeptide der Formel ($I_B$) gemäß Anspruch 1, worin das Symbol $A'_7$ für ein Valin steht.

4. Das folgende Produkt der Formel ($I_A$) gemäß Anspruch 1:
Cys – Asn – Ser –Leu – $Ser_5$ – Thr – Cys – Val – Leu – $Gly_{10}$ – Lys – Leu – Ser – Gln – $Glu_{15}$ – Leu – His – Lys – Leu – $Gln_{20}$ – Thr – Pro – Arg – $Thr_{25}$ – Asp – Val – Gly – Ala – $Gly_{30}$ – Thr – Proamid.

5. Das folgende Produkt der Formel ($I_B$) gemäß Anspruch 1:

$$\overline{\text{CO}-(CH_2)_5-\text{CH}}$$

CO-Asn-Ser-Leu-Ser-Thr$_5$-NH-CH-CO-Val-Leu-Gly-Lys$_{10}$-Leu- ($I_B$)
Ser-Gln-Glu-Leu$_{15}$-His-Lys-Leu-Gln-Thr$_{20}$-Tyr-Pro-Arg-
Thr-Asp$_{25}$-Val-Gly-Ala-Gly-Thr$_{30}$-Proamid .

6. Verfahren zur Herstellung von neuen, in den Ansprüchen 1 bis 5 angegebenen Polypeptiden, dadurch gekennzeichnet, daß man Aminosäuren, Peptide oder deren Kombinationen Kondensationsreaktionen in der Reihenfolge der Aminosäuresequenz der Formeln ($I_A$) und ($I_B$) unterzieht, und daß man bei der Herstellung der Produkte der Formel ($I_B$) die Technik in fester Phase für die Erzielung der Sequenz 10–31 mit der klassischen Technik in flüssiger Phase für die Erzielung der Sequenz 1–9 kombiniert.

7. Als Arzneimittel die in den Ansprüchen 1 bis 5 angegebenen Polypeptide.

8. Pharmazeutische Zusammensetzungen, enthaltend als Wirkstoff ein Arzneimittel gemäß Anspruch 7.

9. Pharmazeutische Zuasmmensetzungen, enthaltend als Wirkstoff ein oder mehrere Polypeptide gemäß den Ansprüchen 1 bis 5 sowie für die parenterale oder orale Verabreichung in Form von Liposomen und die nasale Verabreichung in Form von Zerstäubungsprodukten geeignete Träger.

**Patentansprüche für die Vertragsstaaten: AT, ES**

1. Verfahren zur Herstellung neuer Polypeptide, ausgewählt unter den Produkten der Formel ($I_A$)

Cys–Asn–$A_3$–Leu–Ser–Thr–Cys–$A_8$–Leu–Gly–$A_{11}$–$A_{12}$–$A_{13}$–Gln–$A_{15}$–$A_{16}$–$A_{17}$–Lys–$A_{19}$–$A_{20}$–Thr–$A_{22}$–Pro–$A_{24}$–Thr–$A_{26}$–$A_{27}$–Gly–$A_{29}$–Gly–$A_{31}$–Pro $NH_2$ ($I_A$)

worin
$A_3$=Ser, Gly oder Ala
$A_8$=Val oder Leu
$A_{11}$=Lys oder Thr
$A_{12}$=Leu oder Tyr
$A_{13}$=Ser oder Thr
$A_{15}$=Glu oder Asp
$A_{16}$=Leu oder Phe
$A_{17}$=His oder Asn
$A_{19}$=Leu oder Phe
$A_{20}$=Gln oder His
$A_{22}$=Tyr oder Phe
$A_{24}$=Arg oder Gln
$A_{26}$=Asp, Asn oder Ala
$A_{27}$=Val, Thr oder Ile
$A_{29}$=Ala, Ser oder Val
$A_{31}$=Thr, Val oder Ala
und den Produkten der Formel ($I_B$)

$$\overline{\text{CO}-(CH_2)_5-\text{NHCHCO}}$$

CO-Asn-A'$_2$-Leu-Ser-Thr-NHCHCO-A'$_7$-Leu-Gly-A'$_{10}$-
A'$_{11}$-A'$_{12}$-Gln-A'$_{14}$-A'$_{15}$-A'$_{16}$-Lys-A'$_{18}$-
A'$_{19}$-Thr-A'$_{21}$-Pro-A'$_{23}$-Thr-A'$_{25}$-A'$_{26}$-Gly-
A'$_{28}$-Gly-A'$_{30}$-Pro $NH_2$ ($I_B$)

worin
A'$_2$=Ser, Gly oder Ala
A'$_7$=Val oder Leu
A'$_{10}$=Lys oder Thr
A'$_{11}$=Leu oder Tyr
A'$_{12}$=Ser oder Thr
A'$_{14}$=Glu oder Asp
A'$_{15}$=Leu oder Phe
A'$_{16}$=His oder Asn
A'$_{18}$=Leu oder Phe

A'$_{19}$=Gln oder His
A'$_{21}$=Tyr oder Phe
A'$_{23}$=Arg oder Gln
A'$_{25}$=Asp, Asn oder Ala
A'$_{26}$=Val, Thr oder Ile
A'$_{28}$=Ala, Ser oder Val
A'$_{30}$=Thr, Val oder Ala,
dadurch gekennzeichnet, daß man Aminosäuren, Peptide oder deren Kombinationen Kondensationsreaktionen in der Reihenfolge der Aminosäuresequenz der Formeln ($I_A$) und ($I_B$) unterzieht, und daß man bei der Herstellung der Produkte der Formel ($I_B$) die Technik in fester Phase für die Erzielung der Sequenz 10–31 und die klassische Technik in flüssiger Phase für die Erzielung der Sequenz 1–9 miteinander kombiniert.

2. Verfahren gemäß Anspruch 1, dadurch gekennzeichnet, daß in der Formel ($I_A$) das Symbol $A_8$ für ein Valin steht und daß in der Formel ($I_B$) das Symbol A'$_7$ für ein Valin steht.

3. Verfahren gemäß Anspruch 1 oder 2, dadurch gekennzeichnet, daß die Ausgangsprodukte derart ausgewählt werden, daß man das folgende Produkt der Formel ($I_A$) gemäß Anspruch 1 herstellt:
Cys–Asn–Ser–Leu–Ser$_5$–Thr–Cys–Val–Leu–Gly$_{10}$–Lys–Leu–Ser–Gln–Glu$_{15}$–Leu–His–Lys–Leu–Gln$_{20}$–Thr–Tyr–Pro–Arg–Thr$_{25}$–Asp–Val–Gly–Ala–Gly$_{30}$–Thr–Proamid.

4. Verfahren gemäß Anspruch 1 oder 2, dadurch gekennzeichnet, daß die Ausgangsprodukte derart ausgewählt werden, daß man das folgende Produkt der Formel ($I_B$) gemäß Anspruch 1 herstellt:

$(CH_2)_5$
CO–Asn–Ser–Leu–Ser–Thr$_5$–NH–CH–CO–Val–Leu–Gly–Lys$_{10}$–Leu–
Ser–Gln–Glu–Leu$_{15}$–His–Lys–Leu–Gln–Thr$_{20}$–Tyr–Pro–Arg–
Thr–Asp$_{25}$–Val–Gly–Ala–Gly–Thr$_{30}$–Proamid .

**Patentansprüche für den Vertragsstaat: GR**

1. Verfahren zur Herstellung neuer Polypeptide, ausgewählt unter den Produkten der Formel ($I_A$)
Cys–Asn–$A_3$–Leu–Ser–Thr–Cys–$A_8$–Leu–Gly–$A_{11}$–$A_{12}$–$A_{13}$–Gln–$A_{15}$–$A_{16}$–$A_{17}$–Lys–$A_{19}$–$A_{20}$–Thr–$A_{22}$–Pro–$A_{24}$–Thr–$A_{26}$–$A_{27}$–Gly–$A_{29}$–Gly–$A_{31}$–Pro $NH_2$
worin
$A_3$=Ser, Gly oder Ala
$A_8$=Val oder Leu
$A_{11}$=Lys oder Thr
$A_{12}$=Leu oder Tyr
$A_{13}$=Ser oder Thr
$A_{15}$=Glu oder Asp
$A_{16}$=Leu oder Phe
$A_{17}$=His oder Asn
$A_{19}$=Leu oder Phe
$A_{20}$=Gln oder His
$A_{22}$=Tyr oder Phe
$A_{24}$=Arg oder Gln
$A_{26}$=Asp, Asn oder Ala
$A_{27}$=Val, Thr oder Ile
$A_{29}$=Ala, Ser oder Val
$A_{31}$=Thr, Val oder Ala
und den Produkten der Formel ($I_B$)

$(CH_2)_5$
CO–Asn–A'$_2$–Leu–Ser–Thr–NHCHCO–A'$_7$–Leu–Gly–A'$_{10}$– ($I_B$)
A'$_{11}$–A'$_{12}$–Gln–A'$_{14}$–A'$_{15}$–A'$_{16}$–Lys–A'$_{18}$–
A'$_{19}$–Thr–A'$_{21}$–Pro–A'$_{23}$–Thr–A'$_{25}$–A'$_{26}$–Gly–
A'$_{28}$–Gly–A'$_{30}$–Pro $NH_2$

worin

A'$_{2}$=Ser, Gly oder Ala
A'$_{7}$=Val oder Leu
A'$_{10}$=Lys oder Thr
A'$_{11}$=Leu oder Tyr
A'$_{12}$=Ser oder Thr
A'$_{14}$=Glu oder Asp
A'$_{15}$=Leu oder Phe
A'$_{16}$=His oder Asn
A'$_{18}$=Leu oder Phe
A'$_{19}$=Gln oder His
A'$_{21}$=Tyr oder Phe
A'$_{23}$=Arg oder Gln
A'$_{25}$=Asp, Asn oder Ala
A'$_{26}$=Val, Thr oder Ile
A'$_{28}$=Ala, Ser oder Val
A'$_{30}$=Thr, Val oder Ala,
dadurch gekennzeichnet, daß man Aminosäuren, Peptide oder deren Kombinationen Kondensationsreaktionen in der Reihenfolge der Aminosäuresequenz der Formeln ($I_A$) und ($I_B$) unterzieht, und daß man bei der Herstellung der Produkte der Formel ($I_B$) die Technik in fester Phase für die Erzielung der Sequenz 10–31 und die klassische Technik in flüssiger Phase für die Erzielung der Sequenz 1–9 miteinander kombiniert.

2. Verfahren gemäß Anspruch 1, dadurch gekennzeichnet, daß in der Formel ($I_A$) das Symbol $A_8$ für ein Valin steht und daß in der Formel ($I_B$) das Symbol A'7 für ein Valin steht.

3. Verfahren gemäß Anspruch 1 oder 2, dadurch gekennzeichnet, daß die Ausgangsprodukte derart ausgewählt werden, daß man das folgende Produkt der Formel ($I_A$) gemäß Anspruch 1 hergestellt:
Cys–Asn–Ser–Leu–Ser$_5$–Thr–Cys–Val–Leu–Gly$_{10}$–Lys–Leu–Ser–Gln–Glu$_{15}$–Leu–His–Lys–Leu–Gln$_{20}$–Thr–Tyr–Pro–Arg–Thr$_{25}$–Asp–Val–Gly–Ala–Gly$_{30}$–Thr–Proamid. ($I_A$)

4. Verfahren gemäß Anspruch 1 oder 2, dadurch gekennzeichnet, daß die Ausgangsprodukte derart ausgewählt werden, daß man das folgende Produkt der Formel ($I_B$) gemäß Anspruch 1 herstellt:

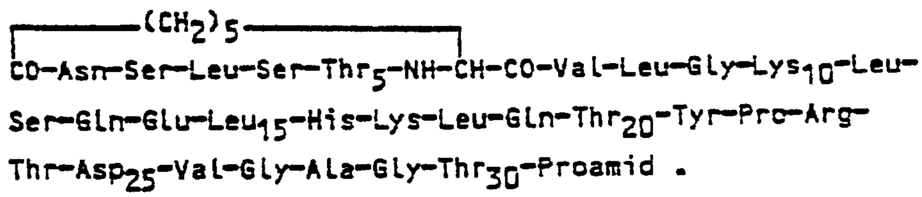
($I_B$)

5. Verfahren zur Herstellung pharmazeutischer Zusammensetzungen, dadurch gekennzeichnet, daß man als Wirkstoff zumindest eines der Polypeptide der Formel ($I_A$) oder ($I_B$) gemäß Anspruch 1 in eine für diese Verwendung bestimmte Form bringt.

6. Verfahren zur Herstellung pharmazeutischer Zusammensetzungen, dadurch gekennzeichnet, daß man als Wirkstoff zumindest eines der Polypeptide der Formel ($I_A$) oder ($I_B$) gemäß Anspruch 3 oder 4 in eine für diese Verwendung bestimmte Form bringt.

**Claims for the contracting states: BE, CH, DE, FR, GB, IT, LI, LU, NL, SE**

1. New polypeptides, characterized in that they are chosen from the group formed by the products of formula ($I_A$):
Cys–Asn–$A_3$–Leu–Ser–Thr–Cys–$A_8$–Leu–Gly–$A_{11}$–$A_{12}$–$A_{13}$–Gln–$A_{15}$–$A_{16}$–$A_{17}$–Lys–$A_{19}$–$A_{20}$–Thr–$A_{22}$–Pro–$A_{24}$–Thr–$A_{26}$–$A_{27}$–Gly–$A_{29}$–Gly–$A_{31}$–Pro $NH_2$ ($I_A$)
where
$A_3$=Ser, Gly or Ala
$A_8$=Val or Leu
$A_{11}$=Lys or Thr
$A_{12}$=Leu or Tyr
$A_{13}$=Ser or Thr
$A_{15}$=Glu or Asp
$A_{16}$=Leu or Phe
$A_{17}$=His or Asn
$A_{19}$=Leu or Phe
$A_{20}$=Gln or His
$A_{22}$=Tyr or Phe

$A_{24}$=Arg or Gln
$A_{26}$=Asp, Asn or Ala
$A_{27}$=Val, Thr or Ile
$A_{29}$=Ala, Ser or Val
$A_{31}$=Thr, Val or Ala
and the products of formula ($I_B$):

┌──── $(CH_2)_5$ ────┐
CO-Asn-A'$_2$-Leu-Ser-Thr-NHCHCO-A'$_7$-Leu-Gly-A'$_{10}$-
A'$_{11}$-A'$_{12}$-Gln-A'$_{14}$-A'$_{15}$-A'$_{16}$-Lys-A'$_{18}$- ($I_B$)
A'-$_{19}$-Thr-A'$_{21}$-Pro-A'$_{23}$-Thr-A'$_{25}$-A'$_{26}$-Gly-
A'$_{28}$-Gly-A'$_{30}$-Pro $NH_2$

where:
A'$_2$=Ser, Gly or Ala
A'$_7$=Val or Leu
A'$_{10}$=Lys or Thr
A'$_{11}$=Leu or Tyr
A'$_{12}$=Ser or Thr
A'$_{14}$=Glu or Asp
A'$_{15}$=Leu or Phe
A'$_{16}$=His or Asn
A'$_{18}$=Leu or Phe
A'$_{19}$=Gln or His
A'$_{21}$=Tyr or Phe
A'$_{23}$=Arg or Gln
A'$_{25}$=Asp, Asn or Ala
A'$_{26}$=Val, Thr or Ile
A'$_{28}$=Ala, Ser or Val
A'$_{30}$=Thr, Val or Ala

2. New polypeptides corresponding to the formula ($I_A$) as defined in claim 1 in which the symbol A8 represents a valine.

3. New polypeptides corresponding to the formula ($I_B$) as defined in claim 1 in which the symbol A'$_7$ represents a valine.

4. The following product of formula ($I_A$) according to claim 1:
Cys–Asn–Ser–Leu–Ser$_5$–Thr–Cys–Val–Leu–Gly$_{10}$–Lys–Leu–Ser–Gln–Glu$_{15}$–Leu–His–Lys–Leu–Gln$_{20}$–Thr–Tyr–Pro–Arg–Thr$_{25}$–Asp–Val–Gly–Ala–Gly$_{30}$–Thr–Proamide.

5. The following product of formula ($I_B$) according to claim 1:

┌──── $(CH_2)_5$ ────┐
CO-Asn-Ser-Leu-Ser-Thr$_5$-NH-CH-CO-Val-Leu-Gly-Lys$_{10}$-Leu-
Ser-Gln-Glu-Leu$_{15}$-His-Lys-Leu-Gln-Thr$_{20}$-Tyr-Pro-Arg- ($I_B$)
Thr-Asp$_{25}$-Val-Gly-Ala-Gly-Thr$_{30}$-Proamide

6. Process for the preparation of the new polypeptides specified in claims 1 to 5, characterized by the fact that amino acids, peptides or their combinations are submitted to condensation reactions in the order of sequence of the amino acids of fomulae ($I_A$) and ($I_B$) and by the fact that during the preparation of the products of formula ($I_B$) the solid phase technique for obtaining the 10–31 sequence and the standard liquid phase technique for obtaining the 1–9 sequence are combined.

7. As medicaments, the polypeptides mentioned in claims 1 to 5.

8. Pharmaceutical compositions containing one medicament according to claim 7 as active principle.

9. The pharmaceutical preparations containing as active principle one or more polypeptides mentioned in claims 1 to 5, as well as appropriate vehicles intended for parenteral administration, oral administration in the form of liposome and nasal administration in the form of a spray.

**Claims for the contracting states: AT, ES**

1. Process for the preparation of new polypeptides chosen from the group formed by the products of formula ($I_A$):

Cys–Asn–$A_3$–Leu–Ser–Thr–Cys–$A_8$–Leu–Gly–$A_{11}$–$A_{12}$–$A_{13}$–Gln–$A_{15}$–$A_{16}$–$A_{17}$–Lys–$A_{19}$–$A_{20}$–Thr–$A_{22}$–Pro–$A_{24}$–Thr–$A_{26}$–$A_{27}$–Gly–$A_{29}$-Gly–$A_{31}$–Pro $NH_2$ ($I_A$)

where:
A3=Ser, Gly or Ala
A8=Val or Leu
A11=Lys or Thr
A12=Leu or Tyr
A13=Ser or Thr
$A_{15}$=Glu or Asp
$A_{16}$=Leu or Phe
$A_{17}$=His or Asn
$A_{19}$=Leu or Phe
$A_{20}$=Gln or His
$A_{22}$=Tyr or Phe
$A_{24}$=Arg or Gln
$A_{26}$=Asp, Asn or Ala
$A_{27}$=Val, Thr or Ile
$A_{29}$=Ala, Ser or Val
$A_{31}$=Thr, Val or Ala
and the products of formula ($I_B$):

$$\overline{\text{CO}\quad(CH_2)_5\quad\text{CH}}$$

CO-Asn-A'$_2$-Leu-Ser-Thr-NHCHCO-A'$_7$-Leu-Gly-A'$_{10}$-
A'$_{11}$-A'$_{12}$-Gln-A'$_{14}$-A'$_{15}$-A'$_{16}$-Lys-A'$_{18}$- ($I_B$)
A'-$_{19}$-Thr-A'$_{21}$-Pro-A'$_{23}$-Thr-A'$_{25}$-A'$_{26}$-Gly-
A'$_{28}$-Gly-A'$_{30}$-Pro $NH_2$

where:
A'$_2$=Ser, Gly or Ala
A'$_7$=Val or Leu
A'$_{10}$=Lys or Thr
A'$_{11}$=Leu or Tyr
A'$_{12}$=Ser or Thr
A'$_{14}$=Glu or Asp
A'$_{15}$=Leu or Phe
A'$_{16}$=His or Asn
A'$_{18}$=Leu or Phe
A'$_{19}$=Gln or His
A'$_{21}$=Tyr or Phe
A'$_{23}$=Arg or Gln
A'$_{25}$=Asp, Asn or Ala
A'$_{26}$=Val, Thr or Ile
A'$_{28}$=Ala, Ser or Val
A'$_{30}$=Thr, Val or Ala
characterized by the fact that the amino acids, peptides or their combinations are submitted to condensation reactions in the order of sequence of the amino acids of formulae ($I_A$) and ($I_B$) and by the fact that during the preparation of the products of formula ($I_B$) the solid phase technique for obtaining the 10–31 sequence and the standard liquid phase technique for obtaining the 1–9 sequence are combined.

2. Process according to claim 1, characterized in that in the formula ($I_A$), the symbol $A_8$ represents a valine and in that in the formula ($I_B$), the symbol A'$_7$ represents a valine.

3. Process according to claim 1 or 2, characterized in that the starting products are chosen in such a way that the product of formula ($I_A$) according to claim 1 is prepared as follows:

Cys–Asn–Ser–Leu–Ser$_5$–Thr–Cys–Val–Leu–Gly–Lys–Leu–Ser–Gln–Glu$_{15}$–Leu–His–Lys–Leu–Gln$_{20}$–Thr–Tyr–Pro–Arg–Thr$_{25}$–Asp–Val–Gly–Ala–Gly$_{30}$–Thr–Proamide.

4. Process according to claim 1 or 2, characterized in that the starting products are chosen in such a way that the product of formula ($I_B$) according to claim 1 is prepared as follows:

$\ulcorner$— $(CH_2)_5$ —$\urcorner$

CO-Asn-Ser-Leu-Ser-$Thr_5$-NH-CH-CO-Val-Leu-Gly-$Lys_{10}$-Leu-
Ser-Gln-Glu-$Leu_{15}$-His-Lys-Leu-Gln-$Thr_{20}$-Tyr-Pro-Arg- $(I_B)$
Thr-$Asp_{25}$-Val-Gly-Ala-Gly-$Thr_{30}$-Proamide

**Claims for the contracting state: GR**

1. Process for the preparation of new polypeptides, chosen from the group formed by the products of formula $(I_A)$:

Cys–Asn–$A_3$–Leu–Ser–Thr–Cys–$A_8$–Leu–Gly–$A_{11}$–$A_{12}$–$A_{13}$–Gln–$A_{15}$–$A_{16}$–$A_{17}$–Lys–$A_{19}$–$A_{20}$–Thr–$A_{22}$–Pro–$A_{24}$–Thr–$A_{26}$–$A_{27}$–Gly–$A_{29}$–Gly–$A_{31}$–Pro $NH_2$

where:

$A_3$=Ser, Gly or Ala
$A_8$=Val or Leu
$A_{11}$=Lys or Thr
$A_{12}$=Leu or Tyr
$A_{13}$=Ser or Thr
A15=Glu or Asp
A16=Leu or Phe
A17=His or Asn
A19=Leu or Phe
A20=Gln or His
A22=Tyr or Phe
A24=Arg or Gln
A26=Asp, Asn or Ala
A27=Val, Thr or Ile
A29=Ala, Ser or Val
A31=Thr, Val or Ala

and the products of formula $(I_B)$:

$\ulcorner$— $(CH_2)_5$ —$\urcorner$

CO-Asn-$A'_2$-Leu-Ser-Thr-NHCHCO-$A'_7$-Leu-Gly-$A'_{10}$-
$A'_{11}$-$A'_{12}$-Gln-$A'_{14}$-$A'_{15}$-$A'_{16}$-Lys-$A'_{18}$- $(I_B)$
$A'$-$_{19}$-Thr-$A'_{21}$-Pro-$A'_{23}$-Thr-$A'_{25}$-$A'_{26}$-Gly-
$A'_{28}$-Gly-$A'_{30}$-Pro $NH_2$

where:

$A'_2$=Ser, Gly or Ala
$A'_7$=Val or Leu
$A'_{10}$=Lys or Thr
$A'_{11}$=Leu or Tyr
$A'_{12}$=Ser or Thr
$A'_{14}$=Glu or Asp
$A'_{15}$=Leu or Phe
$A'_{16}$=His or Asn
$A'_{18}$=Leu or Phe
$A'_{19}$=Gln or His
$A'_{21}$=Tyr or Phe
A'23=Arg or Gln
A'25=Asp, Asn or Ala
A'26=Val, Thr or Ile
A'28=Ala, Ser or Val
A'30=Thr, Val or Ala

characterized by the fact that the amino acids peptides or their combinations are submitted to condensation reactions in the order of sequence of the amino acids of formulae $(I_A)$ and $(I_B)$ and by the fact that during the preparation of the products of formula $(I_B)$ the solid phase technique for obtaining the 1–9 sequence are combined.

2. Process according to claim 1, characterized in that in the formula ($I_A$), the symbol $A_8$ represents a valine and in that in the formula ($I_B$), the symbol $A'_7$ represents a valine.

3. Process according to claim 1 or 2, characterized in that the starting products are chosen in such a way that the product of formula ($I_A$) according to claim is prepared as follows:

Cys–Asn–Ser–Leu–$Ser_5$–Thr–Cys–Val–Leu–$Gly_{10}$–Lys–Leu–Ser–Gln–$Glu_{15}$–Leu–His–Lys–Leu–$Gln_{20}$–Thr–Tyr–Pro–Arg–$Thr_{25}$–Asp–Val–Gly–Ala–$Gly_{30}$–Thr–Proamide.

4. Process according to claim 1 or 2, characterized in that the starting products are chosen in such a way that the product of formula ($I_B$) according to claim 1 is prepared as follows:

$(CH_2)_5$

CO-Asn-Ser-Leu-Ser-$Thr_5$-NH-CH-CO-Val-Leu-Gly-$Lys_{10}$-Leu-
Ser-Gln-Glu-$Leu_{15}$-His-Lys-Leu-Gln-$Thr_{20}$-Tyr-Pro-Arg- ($I_B$)
Thr-$Asp_{25}$-Val-Gly-Ala-Gly-$Thr_{30}$-Proamide.

5. Process for the preparation of pharmaceutical compositions, characterized in that at least one of the polypeptides of formulae ($I_A$) and ($I_B$), is put in as active principle in a form intended for this usage.

6. Process for the preparation of pharmaceutical compositions, characterized in that at least one of the polypeptides of formulae ($I_A$) and ($I_B$) as defined in claims 3 or 4, are put in as active principle in the form intended for this usage.